(19) Europäisches Patentamt
European Patent Office
Office européen des brevets

(11) Publication number: **0 082 569**
**B1**

(12) # EUROPEAN PATENT SPECIFICATION

(45) Date of publication of patent specification: **10.04.85**

(51) Int. Cl.[4]: **C 07 C 43/11**, C 07 C 41/03, B 01 J 31/02

(21) Application number: **82201614.3**

(22) Date of filing: **16.12.82**

(54) **Process for preparing alkanol alkoxylates.**

(30) Priority: **23.12.81 US 334092**

(43) Date of publication of application:
**29.06.83 Bulletin 83/26**

(45) Publication of the grant of the patent:
**10.04.85 Bulletin 85/15**

(84) Designated Contracting States:
**BE CH DE FR GB IT LI NL**

(56) References cited:
**EP-A-0 020 867**
**EP-A-0 026 546**
**EP-A-0 033 359**
**US-A-4 239 917**
**US-A-4 302 613**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Edwards, Charles Lee**
**1722 Ashford Hollow**
**Houston Texas 77077 (US)**

(74) Representative: **Aalbers, Onno et al**
**P.O. Box 302**
**NL-2501 CH The Hague (NL)**

**EP 0 082 569 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to the preparation of alkanol alkoxylates by catalyzed addition reaction of alkylene oxides with alkanols.

Alkanol alkoxylates (or simply alkoxylates, as the terminology is alternatively applied herein) are known materials having utility, for instance, as solvents, surfactants, and chemical intermediates. Alkoxylates in which the alkyl group has a number of carbon atoms in the detergent range, i.e., from about 8 to 20 are common components of commercial cleaning formulations for use in industry and in the home.

Under conventional practice, alkoxylates are typically prepared by the addition reaction of alkylene oxides with alkanols. In the particular case of the preparation of an ethoxylate the addition of a number (n) of ethylene oxide molecules to a single alkanol molecule may be illustrated by the equation

$$R{-}OH + n\ H_2C\overset{O}{\diagup\diagdown}CH_2 \rightarrow R{-}O{+}CH_2{-}CH_2{-}{)_n}H,$$

wherein R is an alkyl group and n is an integer equal to or greater than one.

Alkoxylation reactions between alkylene oxides and alkanols are known to be necessarily carried out in the presence of a catalyst, which may be either of acidic or basic character. Suitable basic catalysts are known to include the soluble basic salts of the alkali metals of Group I of the Periodic Table, e.g., lithium, sodium, potassium, rubidium, and caesium, and the soluble basic salts of certain of the alkaline earth metals of Group II, e.g., barium, strontium, and calcium. With regard to the basic salts of magnesium, however, the most relevant teachings of the art, specifically those of U.S. Patent Nos. 4,239,917, 4,210,764 and 4,233,164 on alkoxylation reactions catalyzed by barium and strontium compounds, indicate that basic magnesium compounds do not effectively promote the alkoxylation of detergent-range alkanols.

Numerous acidic substances, including, broadly, the Lewis acid of Friedel-Crafts catalysts, and specifically the compound magnesium perchlorate, are also known as effective alkoxylation catalysts. However, the use of acid catalysts is undesirable in several processing aspects. For instance, the acidity of the reaction mixture catalyzes side reactions to produce relatively large amounts of polyalkylene glycols. The acid catalysts also react directly with components of the alkoxylation mixture to yield organic derivatives of the acids which for reasons such as toxicity are not acceptable in the alkoxylate product. Furthermore, efficient use of acid catalysts is generally limited to the alkoxylation of secondary alkanols and to the preparation of alkoxylates having an average number of alkylene oxide moieties less than 2 or 3.

It has now surprisingly been found that alkanol alkoxylates may be prepared by the addition reaction between an alkylene oxide reactant and an alkanol reactant carried out in the presence of an alkanol-soluble basic magnesium catalyst together with a specific reaction activator. In the absence of the activator, basic magnesium compounds have been found to be ineffective as catalysts for the alkoxylation.

According to the present invention there is provided a process for preparing alkanol alkoxylates which comprises reacting at least one alkanol having 6 to 30 carbon atoms with at least one alkylene oxide having from 2 to 4 carbon atoms, in the presence of at least one alkanol-soluble basic compound of magnesium and, as reaction activator, at least 2.0 percent by mol, calculated on mols of alkanol, of at least one alkoxylate of at least one $C_1$ to $C_{30}$ alkanol having from 1 to 30 $C_2$ to $C_4$ alkylene oxide moieties.

The process of the invention is preferably carried out at a temperature in the range from 90 to 250°C. A more preferred range is from 130 to 210°C, while a temperature from 150 to 190°C is still more preferred. Considered most preferred is a reaction temperature in the range from 165 to 175°C. Although the pressure under which the alkoxylation reaction is conducted is not critical to the invention, a total pressure in the range from atmospheric pressure to $10^6$ Pa (gauge) (150 psig) is preferred. Under preferred conditions of temperature and pressure, the alkanol reactant and the alkoxylate reaction activator are generally liquid and the alkylene oxide reactant a vapour. The alkoxylation is then most conveniently conducted by contacting gaseous alkylene oxide with a liquid solution of the magnesium compound and the alkoxylate activator in the alkanol. Since, as is known, there is a danger of explosion in alkylene oxides maintained in concentrated form at elevated temperature and pressure, the partial pressure of the alkylene oxide in the vapour phase is preferably limited, for instance, to less than $4\times10^5$ Pa (60 psia), and this reactant is diluted with an inert gas such as nitrogen, for instance, to a vapour phase concentration of 50 percent or less. The reaction can, however, be safely accomplished at greater alkylene oxide concentration, greater total pressure and greater partial pressure of alkylene oxide if suitable precautions, known to the art, are taken to manage the risks of explosion. A total pressure of from 2.8 to $7.6\times10^5$ Pa (gauge) (40 to 110 psig), with an alkylene oxide partial pressure of from 1 to $4\times10^5$ Pa (15 to 60 psia), is particularly preferred, while a total pressure of from 3.5 to $6\times10^5$ Pa (gauge) (50 to 90 psia), with an alkylene oxide partial pressure of from 1.4 to $3.5\times10^5$ Pa (20 to 50 psia) is considered more preferred.

Primary alkanols are particularly preferred alkanols having from 6 to 30 carbon atoms, largely on the basis of rate of the alkoxylation reaction. For reasons relating to the utility of the product alkoxylates in detergent formulations, preference may be expressed for alkanols within

further restricted carbon number ranges. Thus, alkanols in the $C_7$ to $C_{22}$ range are preferred reactants, while those in the $C_8$ to $C_{18}$ range are considered more preferred and those in the $C_{10}$ to $C_{16}$ range most preferred. Still further preference for reason of product utility may be stated for alkanol reactants in which greater than 50 percent, more preferably greater than 70 percent, and most preferably greater than 90 percent of the alkanol molecules are of linear (straight-chain) carbon structure. Mixtures containing a variety of such alkanols, differing, for instance, with respect to carbon number and branching in the carbon chain, are suitable for purposes of the process of the invention and are in most cases preferred because of commercial availability.

The alkylene oxides (epoxides) utilized in the process of the invention include ethylene oxide, propylene oxide, and the 1,2- and 2,3-butylene oxides. Particularly preferred are ethylene oxide and propylene oxide, while the use of ethylene oxide is most preferred. Mixtures of alkylene oxides are suitable, in which case the product of the invention will be a mixed alkoxide.

The alkanol-soluble basic compound of magnesium may be either a magnesium compound which is directly alkanol-soluble or a precursor which interacts with the alkoxylation process reactants to bring the magnesium into solution in a soluble basic form. The magnesium compound is described as soluble in the sense that it must be soluble in the liquid alkanol reactant or a mixture of liquid alkanol reactant and alkoxylate (including both the alkoxylate utilized as activator and that produced in the process) to the extent necessary to promote the desired reaction. Typically, at least 0.1 percent by mol (%m) of the magnesium compound, calculated on mols of total alkanol reactant will be present in the reaction mixture. Preferably, the magnesium compound is present in the reaction mixture in a quantity from 0.2 to 20%m calculated on alkanol, while a quantity from 0.5 to 15%m is more preferred and from 1.5 to 10%m is considered most preferred. As a rule, the rate of the alkoxylation reaction increases as the invention is carried out with increasing quantities of the catalyst. The catalyst is described as basic in the conventional sense, indicating that a hydrolyzed sample of an alkoxylation reaction mixture containing the magnesium compound in a catalytically-effective quantity (e.g., a 10%w solution of the reaction mixture in water) has a pH greater than 7.0. For purposes of the invention, the overall reaction mixture is of basic pH. Examples of specific soluble, basic catalysts suitable for introduction into the reaction mixture include the reaction products of magnesium with various alcohols (e.g. alcoholates such as magnesium alkoxides and phenoxides), as well as ammoniate, amide, thiolate, thiophenoxide and nitride compounds. Preferred for use as catalyst (or catalyst precursor) are the alcoholates, while the alkoxides in particular are considered more preferred. Each alkoxy group of such alkoxides has a carbon number that is preferably in the range from 1 to 30, more preferably in the range from 1 to 6. The most preferred are the $C_1$ to $C_3$ alkoxides i.e. magnesium methoxide, ethoxide and propoxide. Representative of suitable catalyst precursors which are not per se soluble and/or basic but which are converted into soluble, basic compounds in the alkoxylation reaction mixture are the thiocyanates and the carboxylates, such as the formate, acetate, oxalate, citrate, benzoate, laurate, and stearate, and dialkylmagnesium compounds.

In the course of the alkoxylation reaction of the process of the invention, the alkoxylate activator as well as the alkanol reactant reacts with the alkylene oxide reactant. For reasons relating to properties of the process product mixture, the (higher) alkoxylate formed by reaction of the (lower) alkoxylate activator with alkylene oxide is subject to the same preferences, in terms of number of carbon atoms of the alkyl group and number of alkylene oxide moieties, as is the alkoxylate formed during practice of the invention by reaction of the alkanol reactant with the alkylene oxide reactant. (Consideration is given to the contribution of such higher alkoxylates of the activator to properties of the process product mixture because it is in most cases impractical to separate this higher alkoxylate from the principal product formed through alkoxylation of the alkanol reactant.) Thus, the carbon number of the alkyl group is preferably in the range from 6 to 30, more preferably in the range from 8 to 18, and most preferably in the range from 10 to 16. Expressed in another manner, the activator comprises one or more alkoxylation products of alkanols which are preferably in the $C_7$ to $C_{22}$ range, more preferably in the $C_8$ to $C_{18}$ range, and most preferably in the range from $C_{10}$ to $C_{16}$ range. Activators which are the alkoxylate products of alkanols of at least six carbon atoms are also preferred for a favourable influence on alkoxylation reaction rate. It is particularly preferred for the alkyl group of the alkoxylate reaction activator to contain substantially the same number of carbon atoms as does the alkanol reactant. In order to obtain a product mixture in which the higher alkoxylate formed from the activator has an adduct number distribution similar to that of the alkoxylate formed from the alkanol reactant, activators having a relatively low number e.g. 1 to 5, preferably 1 to 3, more preferably 1 to 2, of alkylene oxide moieties are preferred. As indicated above, these preferences relate to desired properties of the product and not to the operability in the use of the materials as reaction activators for purposes of invention.

The source of the activator and the manner in which it is introduced into the reactant and catalyst mixture are not critical to the invention. In like manner to the catalyst, the activator is suitably either added directly to the reaction mixture or formed in situ by interaction of another added substance with the process reactants and/or catalyst. In a particularly preferred mode of

operation, an alkoxylate activator may be formed in situ in the reactants to be utilized by first carrying out a limited alkoxylation reaction between these reactants in the presence of a catalyst (other than magnesium) capable of promoting alkoxylation in the absence of an activator. For instance, a basic alkali metal compound (e.g. sodium or potassium hydroxide), or a basic compound of another alkaline earth metal (e.g., of barium or strontium), or a Lewis acid catalyst (e.g., a halide or boron, antimony, tungsten, iron, nickel, zinc, tin, aluminium, titanium or molybdenum) can be utilized to promote alkoxylation to yield an alkoxylate, suitable as a reaction activator, in the desired quantity. An advantage of such an operation is the formation of an activator having the same carbon number or carbon number distribution in the alkyl group as that of the alkanol reactant, and also having a very low number of alkylene oxide moities per molecule e.g., most preferably from 1 to 2. After formation of the activator, the acid or base catalyst can be neutralized, and, if desired, removed, before the alkoxylation process of the invention is carried out in the presence of the magnesium catalyst. In certain cases, the activator is also suitably formed *in situ* by the introduction, into a mixture of the alkanol reactant, alkylene oxide reactant, and magnesium catalyst, of a reaction initiator other than an alkoxylate activator, for instance, an ether (other than an alkoxylate) or another compound which, in the presence of the magnesium catalyst will stimulate the reaction between alkanol and alkylene oxide reactants to produce a small amount (e.g., 2 to 7%m) of the desired alkanol alkoxylate product. This small degree of alkoxylation of the alkanol reactant inherently produces an alkoxylate activator in the quantity specified herein and the reaction thereafter proceeds in accordance with the invention. In terms of combination of factors, including contamination of the reaction mixture, effectiveness of reaction activation, and overall process efficiency and convenienc, however, introduction into the reaction mixture of such alkoxylates of lesser carbon number or other ethers to produce the specified alkoxylate activator *in situ* is not a preferred method of operation.

For purposes of this invention the reaction activator is preferably present in the reaction mixture in a quantity of at least 3 percent by mol (%m) calculated on mols of alkanol reactant, while quantities of at least 5%m are more preferred, quantities of at least 6%m are still more preferred, and quantities of at least about 7%m are considered most preferred. The minimum amount of activator necessarily present in the reaction mixture for purposes of the invention is believed to be dependent both upon the relative quantity of activator to alkanol and upon the relative quantity of activator to magnesium catalyst. Smaller quantities of activator may be effective with relatively low catalyst concentrations, although with both an increased induction period and a low alkoxylation reaction rate. There is no upper limit upon the quantity of activator which can be employed. The invention is suitably applied to the alkoxylation of both the alkanol reactant component and the alkoxylate activator component in mixtures comprising substantially greater quantities of activator than alkanol, for instance, mixtures prepared by alkoxylation of alkanols in the presence of catalysts other than magnesium compounds.

In terms of processing procedures, the invention is preferably carried out by mixing together the magnesium compound, the liquid alkanol reactant and the liquid alkoxylate activator and then contacting the resulting solution with gaseous alkylene oxide at the specified temperature and pressure. In one preferred mode of operation, the magnesium compound and the activator are first mixed before they are contacted with either of the reactants. If, on the other hand, the magnesium compound and activator are individually introduced into the reaction mixture it is preferred that the alkoxylate activator be put into solution in the liquid reactant phase in the specified quantity before the magnesium compound is mixed with this liquid phase. If the magnesium catalyst compound is added to the alkanol reactant in the absence of the activator, the resulting mixture may form a viscous gel. While the subsequent addition to the gel mixture of the alkoxylate activator acts to break this gel, the gel formation leads to handling problems which can be avoided simply by reversing the order of the addition of the catalyst and activator to the alkanol.

The process of the invention may conveniently be effected as follows.

Following the preparation of a solution of the catalyst and activator in the alkanol in the relative quantities herein specified, the solution is contacted at the desired temperature and pressure with the alkylene oxide reactant. An induction time may be encountered before the alkoxylation reaction commences. Increased quantities of activator function to shorten the induction period. As the alkylene oxide is taken up in the reaction, additional alkylene oxide is added, conveniently at a rate which maintains an approximately constant reaction pressure. Addition of alkylene oxide and its reaction with alkanol to form alkoxylate is continued until the product reaches the average alkylene oxide adduct number desired for the particular process. Generally, although not necessarily, the invention is best utilized in the preparation of alkoxylates having an average number of alkylene oxide moieties in the range of from 1 to 30, expressed in terms of the total mols of alkylene oxide reacted per mol of alkanol. For reasons relating to utility of the alkoxylate in the broadest commercial applications the process is continued to yield a product having a number of alkylene oxide moieties that is preferably between 2 and 20, more preferably between 3 and 15, most preferably between 4 and 12. The time required to

complete a process in accordance with the invention (once it has commenced) in the presence of the specified catalyst and activator, is dependent both upon the degree of alkoxylation that is desired (i.e., upon the average number of alkylene oxide moieties in the product) as well as upon the rate of the alkoxylation reaction. This reaction rate is, in turn, dependent upon such parameters reaction temperature, pressure, and catalyst concentration in the reaction mixture. Under most preferred operating conditions, preparation of an alkoxylate having an average number of alkylene oxide moieties of about 3 can typically be accomplished in ½ to 1 hour, while preparation of a product having an average number of alkylene oxide moieties of about 12 would typically require 4 to 6 hours. These reaction times are merely illustrative and can be substantially reduced by operation at the higher reaction temperatures and/or pressures, although often at the expense of a loss of selectivity in the utilization of the reactants to the desired alkoxylate products. Following the reaction process, the product mixture is usually neutralized by addition of an acid to convert the basic catalyst components to inactive neutral salts. The choice of the acid used is not critical. Examples of suitable acids include acetic acid, sulphuric acid, phosphoric acid, and hydrochloric acid. Acetic acid is generally preferred.

The invention is further illustrated by the following examples.

Example 1

An alkoxylation process in accordance with the invention was conducted in a 300 ml stainless steel autoclave reactor. The alkanol reactant for this process was a "NEODOL" 23 Alcohol (trademark), characterized as a mixture of primary, 80% linear (20% branched) alkanols containing twelve and thirteen carbon atoms (about 40%m $C_{12}$, 60%m $C_{13}$) produced by hydroformylation. Ethylene oxide was employed as the alkylene oxide reactant. The catalyst was magnesium ethoxide.

For use as reaction activator there was prepared from the alkanol reactant an alkoxylate (ethoxylate) of low alkylene (ethylene) oxide adduct number by the following procedure. First, 0.1 gram of 85 percent purity potassium hydroxide (1.51 millimols KOH) was added to 60 grams (309 millimols) of the alkanol reactant in a multineck round bottom flask. The resulting liquid solution was dried to a water content of about 18 ppm by sparging with nitrogen at 130°C for 30 minutes, and then transferred to the autoclave reactor maintained under a nitrogen atmosphere. The reactor was heated to about 170°C and pressurized by addition of nitrogen and the gaseous ethylene oxide to a total pressure of $4.8\times10^5$ Pa (gauge) (70 psig) ($3.8\times10^5$ Pa (55 psia) nitrogen and $2\times10^5$ Pa (30 psia) ethylene oxide). As the ethoxylation reaction commenced, ethylene oxide was added at a rate sufficient to maintain constant total pressure in the system. Over a 15 minute period, 13.6 grams (309 millimols) of ethylene oxide were added. Ethylene oxide addition was then discontinued and the reaction mixture maintained at 170°C for an additional 30 minutes to consume unreacted ethylene oxide. The mixture was cooled to 50°C, transferred under nitrogen to a sample bottle and neutralized with acetic acid to a pH of 6.0. Chromatographic analysis indicated a mixture containing about 46%w of residual alkanol reactant and 54%w of alkoxylate (ethoxylate) molecules having an average adduct number of about 1.85.

For alkoxylation in accordance with the invention, 60 grams of alkanol reactant were blended with 10 grams of the alkoxylate/alkanol mixture prepared as described above. The resulting blend thus contained 64.6 grams of alkanol reactant and, as reaction activator, 5.4 grams of an alkoxylate having an average adduct number of about 1.85. On a molar basis, the blend contained about 6%m activator, calculated on alkanol reactant. This alkanol and activator solution was dried to about 30 ppm water. At 130°C, 2.0 grams of magnesium ethoxide (17.5 millimols) were dissolved in this solution producing a clear, colourless, non-viscous liquid. The catalyst concentration was about 5.2%m, calculated on the 64.6 grams of alkanol reactant. Ethanol resulting from a transalcoholysis reaction of the catalyst was removed from the solution by sparging with nitrogen for 30 minutes at 130°C. After transfer of the solution to the autoclave reactor the system was sealed, heated to 170°C and pressurized to a total pressure of $4.8\times10^5$ Pa (gauge) (70 psig) ($3.8\times10^5$ Pa (55 psia)$N_2$, $2\times10^5$ Pa (30 psia) ethylene oxide reactant). Ethoxylation proceeded without an induction period. A total of 103 grams (2.35 mols) of ethylene oxide was added over three hours, at a rate which maintained the $4.8\times10^5$ Pa (gauge) (70 psig) total pressure in the reactor. The reaction was continued at 130°C for a further 30 minutes, without ethylene oxide addition, to consume unreacted reactant remaining in the system. The product mixture was then cooled, removed from the autoclave and neutralized with acetic acid. Analysis showed that alkoxylation was essentially complete—only 2.5%w residual alkanol remained in the product mixture. The product has an average adduct number of about 6.4 and contained, as the only observed byproduct, about 0.2%w of polyethylene glycols.

Comparative Example A

An alkoxylation process was attempted under the general procedures of Example 1. In this case, however, the process was carried out in the absence of an alkoxylate activator and thus not in accordance with the invention. A mixture of 65 grams of the dried alkanol reactant and 2.0 grams magnesium ethoxide was prepared and sparged with nitrogen for one hour at 130°C. The mixture was then contacted with ethylene oxide in the autoclave reactor maintained under a temperature of 170°C and a total pressure of $4.8\times10^5$ Pa

(gauge) (70 psig) ($3.8\times10^5$ Pa (55 psia) nitrogen and $2\times10^5$ Pa (30 psia) ethylene oxide). No alkoxylation was observed to take place over a period of five hours.

Example 2

In another example of the invention, the mixture containing 46%w of alkanol reactant and 54%w of an ethoxylate of 1.85 average adduct number, prepared in the course of activator preparation for Example 1 was subjected to further ethoxylation. This alkanol/ethoxylate mixture was first dried to 30 ppm water by sparging with nitrogen. Then, at a temperature of 130°C, 2.0 grams (17.5 millimols) of magnesium ethoxide, as catalyst, were dissolved in 68.7 grams of the dried mixture to give a solution containing 31.6 grams of alkanol reactant, 37.1 grams of ethoxylate activator having an average adduct number of 1.85 (an activator concentration of about 84%m, calculated on alkanol), and 2.0 grams catalyst (10.8%m calculated on alkanol reactant).

This alkanol reactant, activator and catalyst mixture was reacted with an ethylene oxide reactant according to the invention, and under the same general procedures as applied in Example 1. The reaction commenced without an induction period. Over a 2.0 hour period, 70 grams of ethylene oxide were introduced into the reactor. The final product mixture had an adduct number of 6.7 and contained only 2.1%w residual alkanol reactant and 0.4%w polyethylene glycols.

Example 3

Another example of the process of the invention was carried out essentially as described in Example 2. In this case, however, magnesium methoxide, instead of magnesium ethoxide, was utilized as catalyst in a quantity of 1.77 grams and about a 5.2%m concentration, calculated on alkanol. The product was found to have an average adduct number of 6.5 and to contain only about 2.1%w of residual alkanol and 0.4%w of polyethylene glycols.

Example 4

To a dried (by sparging) mixture containing 24.9 grams of "NEODOL" 23 alkanol reactant and 70.8 grams (175%m on alkanol reactant) of an ethoxylate activator having an average adduct number of about 2.75, were added 2.0 grams (13.6%m on alkanol) of magnesium ethoxide catalyst. After nitrogen sparging to remove ethanol, the mixture was reacted with ethylene oxide in accordance with the invention and under the general procedures of Example 1. After the alkoxylation reaction commenced (without an induction period), 67 grams of ethylene oxide were added to the reactor over a period of 2.5 hours. Analysis of the alkoxylate product indicated an average alkylene oxide adduct number of 6.0, a residual alkanol content of 2.2%w, and a polyethylene glycol content of 0.8%w.

Example 5

To a dried mixture containing 44.2 grams of the "NEODOL" 23 alkanol reactant and 27.2 grams (43.5%m on alkanol reactant) of an ethoxylate activator having an average adduct number of about 1.84, were added 2.0 (7,7%m on alkanol) of the magnesium ethoxide catalyst. The mixture was sparged and then reacted with ethylene oxide in accordance with the invention and under the general procedures of Example 1. A total of 87 grams of ethylene oxide reactant were added to the reactor over a three hour period. The product mixture has an average ethylene oxide adduct number of about 6.8 and contained only 1.5%w of residual alkanol and 0.05%w of polyethylene glycols.

Example 6

Another example of a process conducted in accordance with the invention is described to illustrate other aspects of the practice of the invention.

For this example, dibutyl magnesium was employed as catalyst (or catalyst precursor) and diethylene glycol, monomethyl ether (an ethoxylate of methanol having an ethylene oxide adduct number of 2) as reaction activator. The catalyst and the activator were premixed—24 millilitres of a 0.5 molar solution of dibutyl magnesium in heptane (containing a total of 12 millimols of dibutyl magnesium) were added dropwise to a solution of 2.88 grams (24 millimols) of the activator in 20 millilitres of tetrahydrofuran. The mixture was refluxed for one hour. Solvent was then evaporated from the mixture and the remainder, a clear, colourless, non-viscous solution, was added to 65 grams (335 millimols) of the "NEODOL" 23 alkanol reactant described in Example 1. The catalyst and activator solution in the alkanol was transferred to the autoclave for reaction with ethylene oxide at 170°C, and $4.8\times10^5$ Pa (gauge) (70 psig) total pressure ($3.8\times10^5$ Pa (55 psia) $N_2$, $2\times10^5$ Pa (30 psia) ethylene oxide). The reaction mixture contained about 3.6%m of the catalyst and about 7.2%m of the activator, both calculated on alkanol reactant. After addition of ethylene oxide to the reactor, no reaction was observed for 1.5 hours. An alkoxylation reaction then proceeded slowly over the next 30 minutes until, after two hours, a good rate of reaction was observed. Two additional hours of reaction converted 88.5% of the alkanol reactant to yield an ethoxylate product having an average adduct number of about 2.7. The product mixture also contained about 8%m, calculated on ethoxylate of $C_{12}$ and $C_{13}$ alkanol, of a roughly 5 mol ethylene oxide adduct of methanol which was produced by ethoxylation of the activator.

**Claims**

1. A process for preparing alkanol alkoxylates which comprises reacting at least one alkanol having 6 to 30 carbon atoms, with at least one alkylene oxide having 2 to 4 carbon atoms,

characterized in that reaction is effected in the presence of at least one alkanol-soluble basic compound of magnesium, and, as reaction activator, at least 2.0 percent by mol, calculated on mols of the alkanol, of at least one alkoxylate of at least one $C_1$ to $C_{30}$ alkanol having from 1 to 30 $C_2$ to $C_4$ alkylene oxide moieties.

2. A process according to claim 1, wherein the basic alkanol-soluble compound of magnesium is selected from the group consisting of alcoholate, ammoniate, amide, thiolate, thiophenoxide, nitride, thiocyanate and carboxylate compounds and dialkylmagnesium compounds.

3. A process according to claim 2, wherein the basic soluble compound of magnesium is selected from the group consisting of alcoholates having from 1 to 30 carbon atoms.

4. A process according to any of claims 1 to 3, wherein the reaction activator is an alkoxylate of at least one $C_6$ to $C_{30}$ alkanol having from 1 to 30 alkylene oxide moieties independently selected from ethylene oxide and propylene oxide.

5. A process according to claim 4, wherein the reaction activator is an alkoxylate of at least one $C_8$ to $C_{18}$ alkanol having from 1 to 5 alkylene oxide moieties.

6. A process according to claim 5, wherein the alkylene oxide moieties in the reaction activator are from 1 to 3 ethylene oxide moieties.

7. A process according to any of claims 1 to 6, wherein the soluble basic magnesium compound is present in an amount of from 0.2 to 20 percent by mol, calculated on mols of the alkanol, and the reaction activator is present in an amount of at least 3 percent by mol, calculated on mols of the alkanol.

8. A process according to claim 7, wherein the amount of the reaction activator is at least 5 percent by mol.

9. A process according to claim 8, wherein the amount of the reaction activator is at least 6 percent by mol.

10. A process according to any of claims 1 to 9, wherein the soluble basic magnesium compound is present in an amount of from 0.5 to 15 percent by mol, calculated on mols of alkanol reactant.

11. A process according to any of claims 1 to 10, wherein reaction is effected at a temperature between 150 and 190°C, the alkanol or alkanols have 10 to 16 carbon atoms, the alkylene oxide is ethylene oxide and the reaction activator is an ethoxylate of at least one $C_{10}$ to $C_{16}$ alkanol having from 1 to 5 ethylene oxide moieties.

**Patentansprüche**

1. Verfahren zur Herstellung von Alkanolalkoxylaten, umfassend die Umsetzung zumindest eines Alkanols mit 6 bis 30 Kohlenstoffatomen, mit zumindest einem Alkylenoxid mit 2 bis 4 Kohlenstoffatomen, dadurch gekennzeichnet, daß die Reaktion in Gegenwart von zumindest einer in Alkanol löslichen basischen Verbindung von Magnesium und als Reaktionsaktivator zumindest 2,0 mol-%, berechnet auf mol des Alkanols, zumindest eines Alkoxylats, zumindest eines $C_1$- bis $C_{30}$-Alkanols mit 1 bis 30 $C_2$- bis $C_4$-Alkylenoxideinheiten durchgeführt wird.

2. Verfahren nach Anspruch 1, wobei die basische in Alkanol lösliche Magnesiumverbindung ausgewählt ist aus der Gruppe bestehend aus Alkoholat-, Ammoniat-, Amid-, Thiolat-, Thiophenoxid-, Nitrid-, Thiocyanat- und Carboxylatverbindungen und Dialkylmagnesiumverbindungen.

3. Verfahren nach Anspruch 2, wobei die basische lösliche Magnesiumverbindung ausgewählt ist aus der Gruppe bestehend aus Alkoholaten mit 1 bis 30 Kohlenstoffatomen.

4. Verfahren nach einem der Ansprüche 1 bis 3, wobei der Reaktionsaktivator ein Alkoxylat mit zumindest einem $C_6$- bis $C_{30}$-Alkanol mit 1 bis 30 Alkylenoxideinheiten, unabhängig voneinander ausgewählt aus Alkylenoxid und Propylenoxid, ist.

5. Verfahren nach Anspruch 4, wobei der Reaktionsaktivator ein Alkoxylat von zumindest einem $C_8$- bis $C_{18}$-Alkanol mit 1 bis 5 Alkylenoxideinheiten ist.

6. Verfahren nach Anspruch 5, wobei die Alkylenoxideinheiten in dem Reaktionsaktivator 1 bis 3 Ethylenoxideinheiten sind.

7. Verfahren nach einem der Ansprüche 1 bis 6, wobei die lösliche basische Magnesiumverbindung in einer Menge von 0,2 bis 20 mol-%, berechnet auf mol des Alkanols, und der Reaktionsaktivator in einer Menge von zumindest 3 mol-%, berechnet auf mol des Alkanols, vorhanden ist.

8. Verfahren nach Anspruch 7, wobei die Menge an Reaktionsaktivator zumindest 5 mol-% beträgt.

9. Verfahren nach Anspruch 8, wobei die Menge an Reaktionsaktivator zumindest 6 mol-% beträgt.

10. Verfahren nach einem der Ansprüche 1 bis 9, wobei die lösliche basische Magnesiumverbindung in einer Menge von 0,5 bis 15 mol-%, berechnet auf mol des Alkanolreaktionsteilnehmers, vorhanden ist.

11. Verfahren nach einem der Ansprüche 1 bis 10, wobei die Reaktion bei einer Temperatur zwischen 150 und 190°C durchgeführt wird, das Alkanol oder die Alkanole 10 bis 16 Kohlenstoffatome enthalten, des Alkylenoxid Ethylenoxid ist und der Reaktionsaktivator ein Ethoxylat von zumindest einem $C_{10}$- bis $C_{16}$-Alkanol mit 1 bis 5 Ethylenoxideinheiten ist.

**Revendications**

1. Procédé de préparation d'alcanols alcoxylés dans lequel on fait réagir au moins un alcanol en $C_6$ à $C_{30}$ avec au moins un alcoylène-oxyde en $C_2$ à $C_4$, caractérisé en ce que la réaction est conduite en présence d'au moins un composé basique de magnésium soluble dans les alcanols, et, comme activateur de réaction, au moins 2,0% molaire, calculé par rapport au nombre de moles de l'alcanol, d'au moins 1 alcoxylate d'au moins un

alcanol en $C_1$ à $C_{30}$ ayant de 1 à 30 fractions alcoylène-oxyde en $C_2$ à $C_4$.

2. Procédé selon la revendication 1, où le compose basique, soluble dans les alcanols, de magnésium est choisi dans le groupe constitué par les composés alcoolate, ammoniate, amide, thiolate, thiophénoxyde, nitrure, thiocyanate et carboxylate et les composés de dialcoyl-magnésium.

3. Procédé selon la revendication 2, où le composé de magnésium basique soluble est choisi dans le groupe constitué par les alcoolates en $C_1$ à $C_{30}$.

4. Procédé selon l'une quelconque des revendications 1 à 3, ou l'activateur de réaction est un alcoxylate d'au moins un alcanol en $C_6$ à $C_{30}$ ayant de 1 à 30 fractions alcoylène-oxyde choisies indépendamment entre l'éthylène-oxyde et le propylène-oxyde.

5. Procédé selon la revendication 4, où l'activateur de réaction est un alcoxylate d'au moins un alcanol en $C_8$ à $C_{18}$ ayant de 1 à 5 fractions alcoylène-oxyde.

6. Procédé selon la revendication 5, où les fractions alcoylène-oxyde dans l'activateur de réaction sont de 1 à 3 fractions éthylene-oxyde.

7. Procédé selon l'une quelconque des revendications 1 à 6, où le composé de magnésium basique soluble est présent en une quantité allant de 0,2 à 20% en moles, calculée d'apres le nombre de moles de l'alcanol, et l'activateur de réaction est présent en une quantité d'au moins 3% molaire, calculée sur la base du nombre de moles de l'alcanol.

8. Procédé selon la revendication 7, où la quantité de l'activateur de réaction est d'au moins 5% molaire.

9. Procédé selon la revendication 8, où la quantité de l'activateur de réaction est d'au moins 6% molaire.

10. Procédé selon l'une quelconque des revendications 1 à 9, où le composé de magnésium basique soluble est présent en une quantité allant de 0,5 à 15% molaire, calculée d'après le nombre de moles du réactif alcanol.

11. Procédé selon l'une quelconque des revendications 1 à 10, où la réaction est conduite à une température comprise entre 150 et 190°C, le ou les alcanol(s) a(ont) de 10 à 16 atomes de carbone, l'alcoylène-oxyde est l'éthylène-oxyde et l'activateur de réaction est un éthoxylate d'au moins un alcanol en $C_{10}$ à $C_{16}$ ayant de 1 à 5 fractions ethylène-oxyde.